# EUROPEAN PATENT APPLICATION

(11) **EP 4 344 614 A1**
(43) Date of publication of application: **03.04.2024**
(21) Application number: 23196386.9
(22) Date of filing: 08.09.2023
(51) Int. Cl.: A61B 3/10, A61B 3/14, A61B 3/15

(54) **THICKNESS MEASURING METHOD AND DEVICE**

(30) Priority: 28.09.2022 KR 20220123381
(71) Applicant: Huvitz Co., Ltd., Anyang-si, Gyeonggi-do 14055 (KR)
(72) Inventor: OH, Jae Seung, 14055 Gyeonggi-do (KR); CHOI, Seon Hwan, 14055 Gyeonggi-do (KR)
(74) Representative: IPAZ

(57) **Abstract**

Disclosed are a method and device for measuring a corneal thickness of an eye to be examined. An embodiment provides a thickness measuring device, including: a measurement light source (10) that irradiates measurement light L0 to a measurement object having a predetermined thickness; a detection camera (12) that is positioned at an angle spaced by a predetermined angle with respect to a traveling path of the measurement light L0 and that detects images of reflected lights L1 and L2 formed by reflection of the measurement light L0 on front and rear surfaces of the measurement object; a focus camera (14) that detects an image of the measurement light L0 formed on the measurement object; and a calculation and control member (20) that simultaneously drives the focus camera (14) and the detection camera (12) to obtain the image of the measurement light L0 formed on the measurement object with the focus camera (14) and to obtain the images of the reflected lights L1 and L2 formed by reflection on the front and rear surfaces of the measurement object with the detection camera (12), then determines whether the measurement object is positioned at the predetermined measurement position with respect to the detection camera (12) from a focus state of the image of the measurement light L0, and then calculates a thickness of the measurement object from the images of the reflected lights L1 and L2.

## Description

### CROSS-REFERENCE TO RELATED APPLICATION

This application claims priority from Korean Patent Application No. 10-2022-0123381, filed on September 28, 2022, which is incorporated herein by reference in its entirety.

### BACKGROUND

### 1. Technical Field

The present invention relates to a thickness measuring method and device, and more particularly, to a method and device that may measure a corneal thickness of an eye to be examined.

### 2. Description of the Related Art

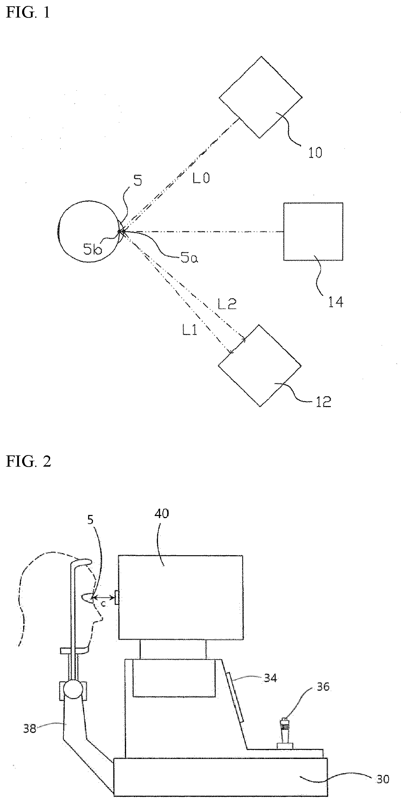

In general, a non-contact method of measuring a corneal thickness of an eye to be examined is performed by emitting measurement light to the eye and detecting reflected light reflected from the cornea of the eye. FIG. 1 is a drawing for explaining a conventional corneal thickness measurement method. As shown in FIG. 1, the conventional corneal thickness measurement device includes a measurement light source 10 for irradiating measurement light L0 to a cornea 5 of the eye to be examined, and a detection camera 12 for detecting images of reflected light L1 and L2 in which the measurement light L0 is reflected from a front surface 5a and a back surface 5b of the cornea 5, respectively, and thereby the images of reflected light L1 and L2 are formed, respectively. The angle between the measurement light L0 and the reflected light L1 and L2 may be 45 to 135 degrees. Since the measurement light L0 is reflected from the front surface 5a and the back surface 5b of the cornea 5, respectively, to form the reflected lights L1 and L2, when the reflected lights L1 and L2, specifically, positions at which the reflected lights L1 and L2 are formed are detected, it is possible to detect the positions of the front surface 5a and the back surface 5b of the cornea 5, and it is possible to calculate a thickness of the cornea 5 from the detected positions of the front surface 5a and the back surface 5b. Here, the distance and position between the cornea 5 and the detection camera 12 must be accurately maintained so that an accurate corneal thickness can be obtained from the positions of the reflected lights L1 and L2 (detection lights). Therefore, as shown in FIG. 1, the image of the measurement light L0 formed on the cornea 5 is detected using a separate focus camera 14. From an image state of the measurement light L0 captured by the focus camera 14, that is, from a focus state, it is checked whether the cornea 5 is positioned at a correct position (distance) with respect to the detection camera 12.

In the corneal thickness measurement described above, an image (focus image) of the measurement light L0 formed on the cornea 5 is first detected using the focus camera 14 and then the images of the reflected lights L1 and L2 which are formed by reflections of the measurement light L0 on the cornea 5 are detected using the detection camera 12. Thus, a predetermined time delay occurs between (i) the acquisition of the focus image and the signal processing by the focus camera 14 and (ii) the acquisition of the reflected light L1 and L2 images and the signal processing by the detection camera 12. When the position of the eye to be examined is changed during the time delay, the positions of the reflected lights L1 and L2 captured by the detection camera 12 become inaccurate, and a correct corneal thickness may not be obtained. That is, when the position of the cornea 5 is confirmed using the focus camera 14 and then the positions of the reflected lights L1 and L2 are detected using the detection camera 12, due to the measurement time difference between them, the position of the cornea 5 may be changed, and thus corneal thickness measurement errors may occur.

### SUMMARY

An object of the present invention is to provide a thickness measuring method and device that may more accurately measure a thickness of a measurement object.

Another object of the present invention is to provide a thickness measuring method and device that may prevent measurement errors by simultaneously obtaining a focus state of measurement light and a measurement signal when measuring a thickness of a measurement object.

An embodiment of the present invention provides a thickness measuring device, including: a measurement light source that irradiates measurement light L0 to a measurement object having a predetermined thickness; a detection camera that is positioned at an angle spaced by a predetermined angle with respect to a traveling path of the measurement light L0 and that detects images of reflected lights L1 and L2 formed by reflection of the measurement light L0 on front and rear surfaces of the measurement object; a focus camera that detects an image of the measurement light L0 formed on the measurement object; and a calculation and control member that simultaneously drives the focus camera and the detection camera to obtain the image of the measurement light L0 formed on the measurement object with the focus camera and to obtain the images of the reflected lights L1 and L2 formed by reflection on the front and rear surfaces of the measurement object with the detection camera, then determines whether the measurement object is positioned at the predetermined measurement position with respect to the detection camera from a focus state of the image of the measurement light L0, and then calculates a thickness of the measurement object from the images of the reflected lights L1 and L2.

Another embodiment of the present invention provides a thickness measuring method, including the steps of: irradiating measurement light L0 to a measurement object having a predetermined thickness to obtain an image of the measurement light L0 formed on the measurement object, and at the same time, obtaining image of the reflected light L1 and L2 formed by reflection of the measurement light L0 on front and rear surfaces of the measurement object; determining whether the measurement object is positioned at a predetermined measurement position from a focus state of the image of the measurement light L0; and calculating a thickness of the measurement object from the image of the reflected lights L1 and L2 formed by the reflection of the measurement light L0 on the front and rear surfaces of the measurement object when the measurement object is positioned at the predetermined measurement position.

According to the thickness measuring method and device according to the present invention, it is possible to reduce or prevent measurement errors by simultaneously obtaining a focus state of measurement light and a measurement signal when measuring a thickness of a measurement object. Thereby, the thickness of the measurement object can be measured more accurately.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a drawing for explaining a conventional corneal thickness measuring method.
FIG. 2 illustrates an example of an ophthalmoscope in which a thickness measuring device according to an embodiment of the present invention may be used.
FIG. 3 illustrates a configuration of a thickness measuring device according to an embodiment of the present invention.
FIG. 4 illustrates an example of integrated image data generated by integrating focus image data and reflected light image data in a corneal thickness measuring device according to an embodiment of the present invention.

### DETAILED DESCRIPTION

Hereinafter, with reference to the accompanying drawings, the present disclosure will be described in detail. In the accompanying drawings, the same reference numerals are denoted to components performing the same or similar functions as those of the prior art.

FIG. 2 illustrates an example of an ophthalmoscope in which a thickness measuring device according to an embodiment of the present invention may be used. The thickness measuring device according to the present invention may be included in an ophthalmoscope such as a tonometer in order to measure a thickness of a cornea of an eye to be examined. As shown in FIG. 2, the ophthalmoscope in which the thickness measuring device according to the present invention is used includes a main body 30 and a thickness measuring device 40 movably installed on the main body 30. A monitor 34 for displaying measurement values, an eye to be examined, and the like, and a movement controller 36 for aligning the thickness measuring device 40 with the eye to be examined are provided on one side of the main body 30. An examinee may place his or her face on a face supporting member 38 provided on the other side of the main body 30. To examine the eye, the thickness measuring device 40 is aligned with the eye. With respect to the eye to be examined, the thickness measuring device 40 can move in three directions (left and right (horizontal), up and down (vertical), and front and back (depth)) by driving three-axis motors or by manual movement. Thereby, the thickness measuring device 40 can be positioned at a predetermined position relative to the eye by manual or electrical actuation.

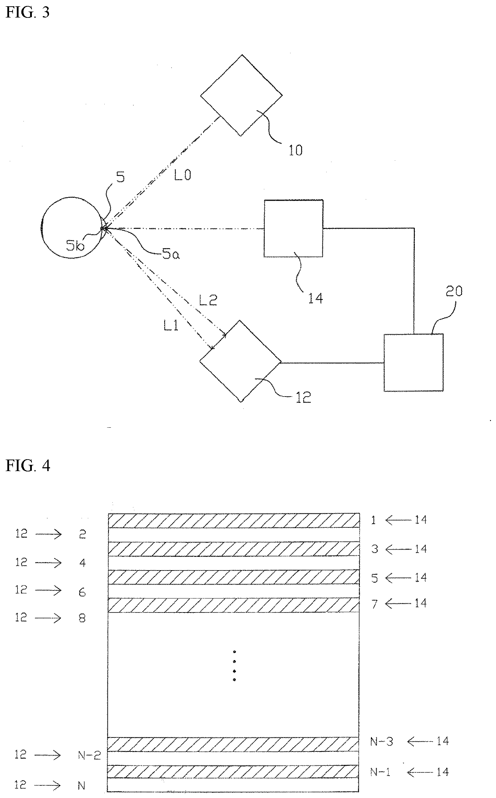

FIG. 3 illustrates a configuration of a thickness measuring device according to an embodiment of the present invention. As shown in FIG. 3, the thickness measuring device 40 according to the embodiment of the present invention includes a measurement light source 10, a detection camera 12, a focus camera 14, and a calculation and control member 20. The measurement light source 10 radiates the measurement light L0 to a measurement object having a predetermined thickness, for example, the cornea 5 of the eye. The measurement light source 10 may be disposed in front of the eye, but as shown in FIG. 3, it may be disposed at a predetermined angle, for example, 30 to 60 degrees (namely, tilted by 30 to 60 degrees), in a vertical or horizontal direction with respect to the eye. The detection camera 12 is positioned at a predetermined angle, for example, 15 to 165 degrees, preferably 45 to 135 degrees, for example, 90 degrees apart from the traveling path of the measurement light L0 to detect images of the reflected lights L1 and L2 formed by reflection of the measurement light L0 on the front and rear surfaces of the measurement object, for example, the front surface 5a and the rear surface 5b of the cornea 5. The focus camera 14 detects the image of the measurement light L0 formed on the measurement object. The focus state of the image of the measurement light L0 is used to determine whether the measurement object is positioned at a predetermined (desired) measurement position (distance) with respect to the detection camera 12. For example, when the measurement light L0 is not well focused on the measurement object and thereby the image of the measurement light L0 is blurred, it is determined that the measurement object is not positioned at a predetermined measurement position with respect to the detection camera 12, for example, is not positioned at a standard measurement distance (c) (see FIG. 2).

The calculation and control member 20 simultaneously drives the focus camera 14 and the detection camera 12 (i) to capture an image (focus image) of the measurement light L0 formed on the measurement object with the focus camera 14, (ii) to capture the images of the reflected lights L1 and L2 formed by reflection from the front and rear surfaces of the measurement object with the detection camera 12, and (iii) determines whether the measurement object is positioned at a predetermined measurement position with respect to the detection camera 12 from the focus state of the image of the measurement light L0. Here, when the measurement object is positioned at a predetermined measurement position with respect to the detection camera 12, the calculation and control member 20 calculates the thickness of the measurement object from the images of the reflected lights L1 and L2 formed by reflection of the measurement light L0 on the front and rear surfaces of the measurement object.

FIG. 4 illustrates, in the corneal thickness measuring device according to the embodiment of the present invention, an example of integrated image data generated by the calculation and control member 20 integrating (i) the image data of the measurement light L0 captured by the focus camera 14 and (ii) the image data of the reflected lights L1 and L2 captured by the detection camera 12. As shown in FIG. 4, the calculation and control member 20 (i) captures image data of the measurement light L0 from the focus camera 14 in units of rows, (ii) captures the image data of the reflected lights L1 and L2 from the detection camera 12 in units of rows, and (iii) then alternately disposes each row of image data of the measurement light L0 and each row of image data of the reflected lights L1 and L2 to form one integrated image data. For example, each row of the image data of the measurement light L0 is disposed in an odd-numbered row of the integrated image data, and each row of the image data of the reflected lights L1 and L2 is disposed in an even-numbered row of the integrated image data. In processing the integrated image data, the calculation and control member 20 extracts data of the odd-numbered row from the integrated image data to obtain the image of the measurement light L0, and determines whether the measurement object is positioned at the correct position with respect to the detection camera 12 from the focus state of the obtained image of the measurement light L0. In this case, when the measurement object is positioned at a predetermined measurement position (c) (a correct position or a standard measurement position, see FIG. 2) with respect to the detection camera 12, the calculation and control member 20 extracts data of the even-numbered row of the integrated image data to obtain the images of the reflected lights L1 and L2 formed by reflection of the measurement light L0 on the front and rear surfaces of the measurement object, and from this, it calculates the thickness of the measurement object. That is, in the embodiment shown in FIG. 4, since the data of the odd-numbered rows includes the image data of the measurement light L0 captured by the focus camera 14 and the data of the even-numbered rows includes the image data of the reflected lights L1 and L2 (detection light) captured by the detection camera 12, the integrated image data captures a focus image and a reflected light image at the same time, and the odd-numbered lines are used to check the distance and position of the measurement object, and at the same time, the thickness of the measurement object may be calculated through the measurement signal in the even-numbered lines. Here, the data of the odd-numbered lines and the even-numbered lines of the integrated image data may be interchanged.

In order to measure the thickness of a measurement object according to the present invention, first, the measurement light L0 is irradiated to a measurement object having a predetermined thickness to obtain an image of the measurement light L0 formed on the measurement object, and at the same time, images of the reflected light L1 and L2 formed by reflection of the measurement light L0 on the front and rear surfaces of the measurement object are obtained. Next, it is determined whether the measurement object is positioned at a predetermined measurement position from the focus state of the image of the measurement light L0. Here, when the measurement object is positioned at a predetermined measurement position, the thickness of the measurement object is calculated from the images of the reflected lights L1 and L2 formed by reflection of the measurement light L0 on the front and rear surfaces of the measurement object.

According to the thickness measuring method (hereinafter, Pachy measurement) and device according to the present invention, when measuring the thickness of the measurement object, it is possible to improve the correlation between the focus state of the measurement light and the measurement signal by simultaneously obtaining (i) the focus image of the measurement light of the measurement object and (ii) the measurement signal image. That is, a focus image of the measurement object is obtained and a measurement signal, that is, an image of the reflected light is simultaneously obtained, and when it is confirmed that the measurement object is present at a predetermined measurement position, thickness can be immediately calculated from the measurement signal image. Therefore, according to the present invention, by simultaneously obtaining a focus image and a measurement signal image, it is possible to eliminate a time delay between the capturing of the focus image and the capturing of the measurement signal image and thereby to improve a measurement accuracy.

Although the present invention has been described with reference to the accompanying drawings and embodiments, the present invention is not limited to the contents shown in the drawings and the above-described embodiments. Reference numerals are indicated in the following claims to aid understanding, but the scope of the following claims is not limited to the contents shown in the reference numerals and drawings, and should be construed to cover all modifications, equivalent configurations, and functions of the embodiments.

## Claims

1. A thickness measuring device (40), comprising:
a measurement light source (10) that irradiates measurement light L0 to a measurement object having a predetermined thickness;
a detection camera (12) that is positioned at an angle spaced by a predetermined angle with respect to a traveling path of the measurement light L0 and that detects images of reflected lights L1 and L2 formed by reflection of the measurement light L0 on front and rear surfaces of the measurement object;
a focus camera (14) that detects an image of the measurement light L0 formed on the measurement object; and
a calculation and control member (20) that simultaneously drives the focus camera (14) and the detection camera (12) to obtain the image of the measurement light L0 formed on the measurement object with the focus camera (14) and to obtain the images of the reflected lights L1 and L2 formed by reflection on the front and rear surfaces of the measurement object with the detection camera (12), then determines whether the measurement object is positioned at the predetermined measurement position with respect to the detection camera (12) from a focus state of the image of the measurement light L0, and then calculates a thickness of the measurement object from the images of the reflected lights L1 and L2.

2. The thickness measuring device (40) according to claim 1, wherein the calculation and control member (20) integrates data of the image of the measurement light L0 obtained with the focus camera (14) and data of the image of the reflected lights L1 and L2 obtained with the detection camera (12) to generate data of an integrated image.

3. The thickness measuring device (40) according to claim 1, wherein the data of the integrated image is formed by (i) obtaining the data of the image of the measurement light L0 with the focus camera (14) in units of rows, (ii) obtaining the data of the images of the reflected lights L1 and L2 with the detection camera (12) in units of rows and (iii) then alternately disposing each row of the image data of the measurement light L0 and each row of the image data of the reflected lights L1 and L2.

4. The thickness measuring device (40) according to claim 1, wherein the measurement object is a cornea of an eye.

5. A thickness measuring method, comprising the steps of:
irradiating measurement light L0 to a measurement object having a predetermined thickness to obtain an image of the measurement light L0 formed on the measurement object, and at the same time, obtaining image of the reflected light L1 and L2 formed by reflection of the measurement light L0 on front and rear surfaces of the measurement object;
determining whether the measurement object is positioned at a predetermined measurement position from a focus state of the image of the measurement light L0; and
calculating a thickness of the measurement object from the image of the reflected lights L1 and L2 formed by the reflection of the measurement light L0 on the front and rear surfaces of the measurement object when the measurement object is positioned at the predetermined measurement position.

6. The thickness measuring method according to claim 5, further comprising the steps of:
obtaining the data of the image of the measurement light L0 in units of rows;
obtaining the data of the image of the reflected lights L1 and L2 in units of rows; and
forming data of an integrated image by alternately disposing (i) each row of the image data of the measurement light L0 and (ii) each row of the image data of the reflected lights L1 and L2.

7. The thickness measuring method according to claim 6, wherein odd-numbered lines of the integrated image data are used to determine a distance and position of the measurement object, and even-numbered lines thereof are used to calculate thickness of the measurement object.
